# EUROPEAN PATENT SPECIFICATION

(11) **EP 2 216 038 B1**
(45) Date of publication and mention of the grant of the patent: **10.08.2016**
(21) Application number: 07845749.6
(22) Date of filing: 30.11.2007
(51) Int. Cl.: A61K 36/725, A61K 36/484, A61K 36/258, A61P 25/24, A61P 25/22

(54) **PHARMACEUTICAL COMPOSITIONS FOR TREATING DEPRESSION AND ANXIETY**
PHARMAZEUTISCHE ZUSAMMENSETZUNGEN ZUR BEHANDLUNG VON DEPRESSION UND ANGSTZUSTÄNDEN
COMPOSITIONS PHARMACEUTIQUES DESTINÉES À TRAITER LA DÉPRESSION ET L'ANXIÉTÉ

(43) Date of publication of application: 11.08.2010
(73) Proprietor: Chi, Yu-Fen, Taiwan (CN); Zhang, Zuoguang, Beijing 100000 (CN)
(72) Inventor: ZHANG, Zuoguang, Beijing 100000 (CN)
(74) Representative: Isarpatent
(86) International application number: PCT/CN2007/003386
(87) International publication number: WO 2009/070915

(56) References cited:
- CN-A- 1 836 687
- US-A- 6 083 932
- SHORES M M ET AL: "The relationship between anxiety and depression: A clinical comparison of generalized anxiety disorder, dysthymic disorder, panic disorder, and major depressive disorder", COMPREHENSIVE PSYCHIATRY, GRUNE AND STRATTON, ORLANDO, FL, US, vol. 33, no. 4, 1 July 1992 (1992-07-01), pages 237-244, XP022965650, ISSN: 0010-440X, DOI: DOI:10.1016/0010-440X(92)90047-T [retrieved on 1992-07-01]

## Description

The present invention relates to a pharmaceutical composition or the health food manufactured from the raw materials including ginsenoside Rg1 and Rb1, glycyrrhizic acid and optionally jujuba cyclic adenosine monophosphate (jujuba cAMP) as defined in claims 12-14 and its use in treating depression and anxiety disorder as defined in claims 1-6. The present invention also relates to a pharmaceutical composition or the health food for use in treating depression and anxiety disorder as defined in claims 7-11. It has definite functions and components, obvious curative effect, fewer side effects and high safety for long-term usage.

Psychological disorders are caused by brain dysfunction and afflicted individuals show abnormalities in awareness, thought, emotion, behavior, will and intelligence, etc. Psychological disorders occupy four in ten diseases, causing the most serious load in society. People are now paying more and more attention to psychological dysfunction in the course of social development. The medical community and the society as a whole are urgently seeking psychological medicine to combat psychological disorders. Anxiety disorder and depression are the most common psychological dysfunction, with anti-anxiety and anti-depression pharmaceuticals being the main method of treatment.

Anxiety disorder is a psychological disease manifest mainly as anxious emotion. The main characteristics are breakout or continuous anxious emotions such as anxiety, catatonia and fear, etc., in combination with syndromes such as autonomic nerves disturbance, muscle rigidity and exercise disturbance, etc. Since Sigmund Freud had separated anxiety disorder from neurasthenia, scholars around the world have begun large-scale studies on anxiety disorder and have accumulated a large amount of data. According to modem medical research, the etiology of anxiety disorder includes defects in psychological anatomy, neurotransmitter/modulator-receptor and neuro-endocrine system, etc.

The current mainstream anti-anxiety medicine is benzodiazepine the mechanism of which is to modulate the activity of the inhibitory neurotransmitter, gamma-aminobutyric acid (GABA), to reduce and relieve the symptoms. However, benzodiazepine has many side effects including insomnolence, allergy, muscle pain, weakness, nausea, exercise dysfunction, blurred vision, weariness, disturbance and delusion, etc.

Depression is a common disease. World Health Organization (WHO) published, "The incidence of depression in the world is about 11%. At present, there are about 340 million psychological depressed patients in the world, and the number is increasing." The investigation is found that depression will be increased to be the number two common disease in the world from now on to 20 years later."

The current mainstream anti-depression pharmaceuticals are Prozac, Paxil and Zoloft, etc., which belong to selective serotonin reuptake inhibitor (SSRI), serotonin-norepinephrine reuptake inhibitor (SNRI), and norepinephrine and dopamine reuptake inhibitor (NDRI), inhibiting the uptake of 5-hydroxytryptamine (5-HT), norepinephrine (NE) and dopamine (DA). The mechanism by which these anti-depression pharmaceuticals function is by increasing the amount of human neurotransmitters such as 5-HT so as to alleviate the symptoms of depression. However, the marketed anti-depression pharmaceuticals have side effects with different levels, such as increased suicide rate, headache, giddiness, vertigo, insomnolence, hypersomnia, tinnitus, thirsty, apocleisis, orexis, increased body weight, increased blood pressure, stomach upset, regurgitation nausea, emesis, dyspepsia, diarrhea, constipation, leg pain, skin rash, dither, convulsions, hyperhidrosis, edema, sexual appetite and impotence, etc.

In recent years, anti-depression pharmaceuticals, such as Prozac, etc., had become a serious social problem. In 2004, the Food and Drug Administration (FDA) of the United States further mandated the pharmaceutical companies to revise product labels to clearly state the side effects and cautions in the instructions of 32 major anti-depression pharmaceuticals in the market, and emphasized to physicians and nurses that these pharmaceuticals might increase children's and adolescents' suicide rates. However, many depression patients having therapeutic condition and therapeutic aspiration stop or refuse their therapies due to worries about or inability to withstand many side effects of the present anti-depression pharmaceuticals. In light of the current situation, the search for a new generation of pharmaceuticals with fewer side effects, high safety for long-term usage and more pronounced/potent anti-depression and anti-anxiety qualities has become the center of attention of the entire pharmaceutical world.

US 6,083,932 describes a pharmaceutical composition comprising a total saponin fraction which is about 20 to 50% by weight of the ginseng extract.

Shores M. et al, Comprehensive Psychiatry, vo. 33, no.4, 2237 - 244 describe generally the relationship between anxiety and depression.

CN 1 836 687 describes pharmaceutical compositions comprising 4 - 18 parts of a ginseng and 3 - 14 parts of a liquorice with or without 3 - 14 parts of a jujuba for use in the treatment of depression.

It is therefore attempted by the applicant to deal with the above situation encountered in the prior art.

### SUMMARY OF THE INVENTION

The purpose of the present invention is to provide a pharmaceutical composition or a health food to overcome the insufficiency of the currently available treatments for depression and anxiety disorder. The pharmaceutical composition or a health food is manufactured from the raw materials including ginsenoside Rg1 and Rb1, glycyrrhizic acid and optionally jujuba cAMP for treating depression and anxiety disorder. In particular, the new technical scheme offering definite functions and components, obvious curative effect, fewer side effects and high safety for long-term usage is provided. More specifically, a pharmaceutical composition is disclosed for use in the treatment of a depression and an anxiety disorder, characterized by comprising:
2 ~ 25 parts by weight of a ginsenoside having an Rg1 and an Rb1; and
3 ~ 46 parts by weight of a glycyrrhizically related acid selected from the group consisting of glycyrrhizic acid, glycyrrhetinic acid and a combination thereof.

The resolving scheme of the pharmaceutical of the present invention is the result endeavored by the inventor in accordance with the pathological and pharmacological theories of modem medicine for treating depression and anxiety disorder. The scheme, employing two or three raw materials including ginseng, liquorice and optionally jujuba, is developed based on the pathological and pharmacological theories of modem medicine for treating anxiety; specifically, it integrates past pharmaceutical-targeted research with the recent knowledge developed in post-receptor mechanism. Ginsenoside from ginseng has adenylate cyclase (AC) activity to stimulate cAMP synthesis and cAMP phosphodiesterase (CAPD) inhibitory activity to reduce cAMP breakdown; glycyrrhizic acid (and glycyrrhetinic acid) from liquorice are strong inhibitors of CAPD. Ginsenoside Rg1 and Rb1 and glycyrrhizic acid when paired and used collectively further increase the concentration and activity of cAMP and protein kinase A (PKA) respectively in the organism. The increasing concentration and activity of cAMP can (1) increase the synthesis and release of the neurotransmitter such as norepinephrine, etc., (2) increase the expression of the brain-derived neurotrophic factor (BDNF), and (3) inhibit the hyperactivity of the hypothalamic-pituitary-adrenal (HPA) axis and the secretion of glucocorticoid, so as to achieve the significant anti-depression function. The increasing concentration and activity of PKA can magnify the inhibition function of GABA on neurons, so as to achieve the significant anti-anxiety function. In addition, jujuba cAMP being the extrinsic non-hydrolyzable cAMP can participate in the metastasis of cAMP in the organism, simulate the enzyme function and increase the expression of cAMP and PKA in the organism, so as to achieve the anti-depression and anti-anxiety functions. Therefore, ginsenoside Rg1 and Rb1 are paired with glycyrrhizic acid and optionally jujuba cAMP; these raw materials are expected to act collectively to further increase the anti-depression effect and the anti-anxiety effect of the present invention. Ginseng, liquorice and jujuba are commonly used pharmaceutical materials and food in Chinese medicine and have been used in dietary nourishing medicinal meals for several thousand years. In this long clinical and dietary history, the safety and efficacy of combined use of ginseng, liquorice and jujuba have been sufficiently proven. The inventor's research and experimental results have shown that if these three pharmaceutical materials are only normally decocted and extracted to obtain the extract, the extract does not have significant anti-depression and anti-anxiety effects compared with the mainstream anti-depression and anti-anxiety pharmaceuticals of the present technology. However, upon further purification of the extract of these three pharmaceutical materials to increase the concentration of the effective components containing ginsenoside Rg1 and Rb1, glycyrrhizic acid and optionally jujuba cAMP, etc., as described in this invention, a pharmaceutical composition with significant anti-depression and anti-anxiety function is obtained. The experimental result has proven that the present invention has significant anti-depression and anti-anxiety effects compared with the mainstream pharmaceuticals, Paroxetine and Diazepam, for treating depression and anxiety disorder respectively. The debris resulting from the three pharmaceutical materials after extraction and purification was also collected and tested in animal experiments. Although the debris contains a trace amount of ginsenoside Rg1 and Rb1, glycyrrhizic acid and jujuba cAMP, it does not show significant anti-depression and anti-anxiety functions in the animal experiments. Importantly, taking ginseng, liquorice and jujuba would not generate the side effects of the mainstream anti-depression and anti-anxiety pharmaceuticals of the present technology. Patients will not need worry about the side effects so they will not cease or refuse the pharmaceutical therapy forever. Therefore, the inventor submits that ginsenoside Rg1 and Rb1, glycyrrhizic acid and optionally jujuba cAMP are the raw materials for manufacturing the pharmaceutical composition or the health food for treating depression and anxiety disorder. In particular, the new technical scheme offers definite functions and components, high safety for long-term usage without side effects, substantially improving the drawbacks generated in the prior art.

Glycyrrhetinic acid has higher liposolubility than glycyrrhizic acid and can easily enter the brain through the blood-brain barrier. Since the glycyrrhizic acid is converted to glycyrrhetinic acid in the human body with almost 100% efficiency, the inhibition of CAPD by glycyrrhizic acid is proceeded by transforming glycyrrhizic acid to glycyrrhetinic acid in the body. Accordingly, glycyrrhizic acid or glycyrrhetinic acid can be the raw material for manufacturing the pharmaceutical composition of the present invention.

In accordance with one aspect of the present invention, a pharmaceutical composition for use in treating at least one of a depression and an anxiety disorder is provided. The pharmaceutical composition includes: ginsenoside Rg1 and Rb1; the glycyrrhizically related acid being one selected from a group consisting of glycyrrhizic acid, glycyrrhetinic acid and a combination thereof; and optionally jujuba cAMP; all of these components are present in the composition in the amounts as specified in the claims.

The pharmaceutical composition includes 2 ~ 25 parts by weight of ginsenoside, 3 ~ 46 parts by weight of the glycyrrhizically related acid and optionally 0.002 ~ 0.4 parts by weight of jujuba cAMP.

Preferably, the pharmaceutical composition includes 4 ~ 12 parts by weight of ginsenoside, 5 ~ 15 parts by weight of the glycyrrhizically related acid and optionally 0.01 ~ 0.08 parts by weight of jujuba cAMP.

Preferably, ginsenoside is extracted from ginseng, the glycyrrhizically related acid is extracted from liquorice, and jujuba cAMP is extracted from jujuba.

Preferably, jujuba is extracted for obtaining a first extract having a first jujuba cAMP concentration, the first extract is further extracted for obtaining a second extract having a second jujuba cAMP concentration, the second jujuba cAMP concentration is higher than the first jujuba cAMP concentration, and the second extract is being a raw material in the pharmaceutical composition.

In accordance with another aspect of the present invention, a pharmaceutical composition preferably used for treating a depression and an anxiety disorder is provided. The pharmaceutical composition includes: ginsenoside; and a glycyrrhizically related acid being one selected from the group consisting of glycyrrhizic acid, glycyrrhetinic acid and a combination thereof; all of these components are present in the composition in the amounts as specified in the claims.

Specifically, the pharmaceutical composition comprises 2 ~ 25 parts by weight of a ginsenoside having an Rg1 and an Rb1, and 3 ~ 46 parts by weight of a glycyrrhizically related acid selected from the group consisting of glycyrrhizic acid, glycyrrhetinic acid and a combination thereof.

Preferably, the pharmaceutical composition includes 4 ~ 12 parts by weight of ginsenoside and 5 ~ 15 parts by weight of the glycyrrhizically related acid.

In accordance with another aspect of the present invention, a pharmaceutical composition for use in the treatment of a depression and an anxiety disorder is provided. The pharmaceutical composition includes ginseng, liquorice and optionally jujuba; all of these components are present in the composition in the amounts as specified in the claims.

The pharmaceutical composition includes 4 ~ 60 parts by weight of ginseng, 2 ~ 30 parts by weight of liquorice and optionally 2 ~ 40 parts by weight of jujuba.

Preferably, the pharmaceutical composition includes 10 ~ 28 parts by weight of ginseng, 5 ~ 14 parts by weight of liquorice and optionally 4 ~ 18 parts by weight of jujuba.

Preferably, the pharmaceutical composition further includes at least one of a pharmacologically acceptable carrier and an additive.

Preferably, the pharmaceutical composition has a dosage form selected from a group consisting of a tablet, a capsule, a powder, a pill, a dust, a solution, a microcapsule, a suspension, an emulsion, a particle, a dropping pill and a roll.

Preferably, the pharmaceutical composition is manufactured as one of a health food and a nutrient supplement.

Further, a preparation method of a pharmaceutical composition containing jujuba cAMP for treating a depression and an anxiety disorder is disclosed but not claimed per se. The preparation method includes steps of: (a) extracting jujuba for obtaining a first extract having a first jujuba cAMP concentration; and (b) purifying the first extract for obtaining a second extract having a second jujuba cAMP concentration. The second jujuba cAMP concentration is higher than the first jujuba cAMP concentration.

Preferably, the step (b) is processed by chromatographing the first extract with a macroporous resin bound with an aldehyde group.

Preferably, the step (b) further includes steps of: (b1) chromatographing the first extract with an OU-2 macrosporous resin bound with the aldehyde group; and (b2) chromatographing the first extract with an ME-2 macroporous resin bound with the aldehyde group.

A method for preparing jujuba cAMP is disclosed but not claimed per se. The method includes steps of: (a) extracting jujuba for obtaining a first extract; and (b) chromatographing the first extract with a macroporous resin having an aldehyde group bound thereon.

The above objectives and advantages of the present invention will become more readily apparent to those ordinarily skilled in the art after reviewing the following detailed descriptions and accompanying drawings, in which:
Fig. 1 is a flowchart showing a preparation method of a pharmaceutical composition in accordance with a first preferred embodiment of the present invention;
Fig. 2 is a flowchart showing a preparation method of a pharmaceutical composition in accordance with a second preferred embodiment of the present invention;
Fig. 3 is a flowchart showing a preparation method of a pharmaceutical composition in accordance with a third preferred embodiment of the present invention;
Fig. 4 is a flowchart showing a preparation method of a pharmaceutical composition in accordance with a fourth preferred embodiment of the present invention;
Fig. 5 is a flowchart showing a preparation method of a pharmaceutical composition in accordance with a fifth preferred embodiment of the present invention; and
Fig. 6 is a flowchart showing a preparation method of a pharmaceutical composition in accordance with a sixth preferred embodiment of the present invention.

The present invention will now be described more specifically with reference to the following embodiments.

In order to accomplish the purpose of the present invention, the technical schemes of the present invention are particularly provided as follows.

A pharmaceutical composition for treating depression and/or anxiety disorder is disclosed in the present invention, and the pharmaceutical composition is manufactured by including the raw materials of ginsenoside Rg1 and Rb1, glycyrrhizic acid and/or jujuba cAMP.

### Example 1:

The pharmaceutical composition for treating depression and anxiety disorder is manufactured from the raw materials including ginseng and liquorice.

### Example 2:

The pharmaceutical composition for treating depression and anxiety disorder is manufactured from the raw materials including 4 ~ 60 parts by weight of ginseng and 2 ~ 30 parts by weight of liquorice.

### Example 3:

The pharmaceutical composition for treating depression and anxiety disorder is manufactured from the raw materials including 10 ~ 28 parts by weight of ginseng and 5 ~ 14 parts by weight of liquorice.

### Example 4:

The pharmaceutical composition for treating depression and anxiety disorder is manufactured from the raw materials including ginseng, liquorice and jujuba.

### Example 5:

The pharmaceutical composition for treating depression and anxiety disorder is manufactured from the raw materials including 4 ~ 60 parts by weight of ginseng, 2 ~ 30 parts by weight of liquorice and 2 ~ 40 parts by weight of jujuba.

### Example 6:

The pharmaceutical composition for treating depression and anxiety disorder is manufactured from the raw materials including 10 ~ 28 parts by weight of ginseng, 5 ~ 14 parts by weight of liquorice and 4 ~ 18 parts by weight of jujuba.

### Example 7:

The pharmaceutical composition for treating depression and anxiety disorder is manufactured from the raw materials including ginsenoside Rg1 and Rb1, and glycyrrhizic acid (or glycyrrhetinic acid).

### Example 8:

The pharmaceutical composition of the present invention is manufactured from the raw materials including a total of 2 - 25 parts by weight of ginsenoside Rg1 and Rb1, and 3 ~ 46 parts by weight of glycyrrhizic acid (or glycyrrhetinic acid).

### Example 9:

The pharmaceutical composition of the present invention is manufactured from the raw materials including a total of 4 ~ 12 parts by weight of ginsenoside Rg1 and Rb1, and 5 ~15 parts by weight of glycyrrhizic acid (or glycyrrhetinic acid).

### Example 10:

The pharmaceutical composition of the present invention is manufactured from the raw materials including the ginseng extract with the above-mentioned parts by weight of ginsenoside Rg1 and Rb1, and the liquorice extract with the above-mentioned parts by weight of glycyrrhizic acid.

### Example 11:

The pharmaceutical composition of the present invention is manufactured from the raw materials including ginsenoside Rg1 and Rb1, glycyrrhizic acid (or glycyrrhetinic acid) and jujuba cAMP.

### Example 12:

The pharmaceutical composition of the present invention is manufactured from the raw materials including a total of 2 ~ 25 parts by weight of ginsenoside Rg1 and Rb1, 3 ~ 46 parts by weight of glycyrrhizic acid (or glycyrrhetinic acid) and 0.002 ~ 0.4 parts by weight of jujuba cAMP.

### Example 13:

The pharmaceutical composition of the present invention is manufactured from the raw materials including a total of 4 ~ 12 parts by weight of ginsenoside Rg1 and Rb1, and 5 ~ 15 parts by weight of glycyrrhizic acid (or glycyrrhetinic acid) and 0.01 ~ 0.08 parts by weight of jujuba cAMP.

### Example 14:

The pharmaceutical composition of the present invention is manufactured from the raw materials including the ginseng extract with the above-mentioned parts by weight of ginsenoside Rg1 and Rb1, the liquorice extract with the above-mentioned parts by weight of glycyrrhizic acid and the jujuba extract with the above-mentioned parts by weight of jujuba cAMP.

### Example 15:

The pharmaceutical composition of the present invention is provided, wherein the raw material having jujuba cAMP is the second extract described as follows. First, jujuba is extracted for obtaining the first extract, then the first extract is further extracted for obtaining the second extract, wherein the jujuba cAMP concentration of the second extract is higher than that of the first extract.

### Example 16:

The pharmaceutical composition of the present invention is provided, wherein the preparation method of the raw material containing jujuba cAMP includes the steps of:
(a) extracting jujuba for obtaining the first extract; and
(b) extracting the first extract for obtaining the second extract, and the jujuba cAMP concentration of the second extract is higher than that of the first extract.

### Example 17:

The above-mentioned preparation method is provided, wherein the step (b) is processed by chromatographing, absorbing and separating jujuba cAMP of the first extract with the macroporous resin bound with the aldehyde group.

### Example 18:

The above-mentioned preparation method is provided, wherein the step (b) is processed by chromatographing, absorbing and separating jujuba cAMP of the first extract with the OU-2 macroporous resin bound with the aldehyde group.

### Example 19:

The above-mentioned preparation method is provided, wherein the step (b) is further processed by chromatographing, absorbing and separating jujuba cAMP of the first extract with the ME-2 macroporous resin bound with the aldehyde group.

### Example 20:

The pharmaceutical composition of the present invention can include the pharmacologically acceptable carriers, additives and the combination thereof.

### Example 21:

The pharmaceutical composition of the present invention can be manufactured as a dosage form, and the dosage forms is selected from any one of tablet, capsule, powder, pill, dust, solution, microcapsule, suspension, emulsion, particle, dropping pill, roll and the pharmacologically oral pharmaceutical dosage form.

### Example 22:

The pharmaceutical composition of the present invention can be manufactured as pharmaceuticals, health food and nutrient supplements for treating depression and anxiety disorder.

In order to accomplish the purpose of the present invention, the preparation methods of the pharmaceutical composition are described as follows.

### Method 1:

The pharmaceutical composition of the present invention for treating depression and anxiety disorder is manufactured from the raw materials of 4 ~ 60 parts by weight of ginseng and 2 ~ 30 parts by weight of liquorice, and the raw materials thereof are extracted and purified for obtaining the extract having ginsenoside Rg1 and Rb1, and glycyrrhizic acid.

### Method 2:

The pharmaceutical composition of the present invention for treating depression and anxiety disorder is manufactured from the raw materials having 10 ~ 28 parts by weight of ginseng and 5 ~ 14 parts by weight of liquorice, and the raw materials thereof are extracted and purified for obtaining the extract having ginsenoside Rg1 and Rb1, and glycyrrhizic acid.

### Method 3:

The pharmaceutical composition of the present invention for treating depression and anxiety disorder is manufactured from the raw materials having 4 ~ 60 parts by weight of ginseng, 2 ~ 30 parts by weight of liquorice and 2 ~ 40 parts by weight of jujuba, and the raw materials thereof are extracted and purified for obtaining the extract having ginsenoside Rg1 and Rb1, glycyrrhizic acid and jujuba cAMP.

### Method 4:

The pharmaceutical composition of the present invention for treating depression and anxiety disorder is manufactured from the raw materials having 10 ~ 28 parts by weight of ginseng, 5 ~ 14 parts by weight of liquorice and 4 ~ 18 parts by weight of jujuba, and the raw materials thereof are extracted and purified for obtaining the extract having ginsenoside Rg1 and Rb1, glycyrrhizic acid and jujuba cAMP.

### Method 5:

The pharmaceutical composition of the present invention for treating depression and anxiety disorder is manufactured from the raw materials of the extract having ginsenoside Rg1 and Rb1 extracted and purified from ginseng, and having glycyrrhizic acid extracted and purified from liquorice, or from the prepared raw materials having ginsenoside Rg1 and Rb1, and glycyrrhizic acid (or glycyrrhetinic acid), all components in the amount as described above and in the claims.

### Method 6:

The pharmaceutical composition of the present invention is manufactured from the raw materials of a total of 2 ~ 25 parts by weight of ginsenoside Rg1 and Rb1, and 3 ~ 46 parts by weight of glycyrrhizic acid (or glycyrrhetinic acid).

### Method 7:

The pharmaceutical composition of the present invention is manufactured from the raw materials of a total of 4 ~ 12 parts by weight of ginsenoside Rg1 and Rb1, and 5 ~ 15 parts by weight of glycyrrhizic acid (or glycyrrhetinic acid).

### Method 8:

The pharmaceutical composition of the present invention for treating depression and anxiety disorder is manufactured from the raw materials of the extract having ginsenoside Rg1 and Rb1 extracted and purified from ginseng, glycyrrhizic acid extracted and purified from liquorice, and jujuba cAMP extracted and purified from jujuba, or from the prepared raw materials of the extract having ginsenoside Rg1 and Rb1, glycyrrhizic acid (or glycyrrhetinic acid) and jujuba cAMP, all components in the amount as described above and in the claims.

### Method 9:

The pharmaceutical composition of the present invention is manufactured from the raw materials of a total of 2 ~ 25 parts by weight of ginsenoside Rg1 and Rb1, 3 ~ 46 parts by weight of glycyrrhizic acid (or glycyrrhetinic acid) and 0.002 ~ 0.4 parts by weight of jujuba cAMP.

### Method 10:

The pharmaceutical composition of the present invention is manufactured from the raw materials of a total of 4 ~ 12 parts by weight of ginsenoside Rg1 and Rb1, 5 ~ 15 parts by weight of glycyrrhizic acid (or glycyrrhetinic acid) and 0.01 ~ 0.08 parts by weight of jujuba cAMP.

### Method 11:

The pharmaceutical composition of the present invention is provided, wherein the preparation method of the raw material containing jujuba cAMP includes the steps of:
(a) extracting jujuba for obtaining the first extract; and
(b) purifying the first extract for obtaining the second extract, and the jujuba cAMP concentration of the second extract is higher than that of the first extract.

### Method 12:

The above-mentioned preparation method is provided, wherein the step (b) is processed by chromatographing, absorbing and separating jujuba cAMP of the first extract with the macroporous resin bound with the aldehyde group.

### Method 13:

The above-mentioned preparation method is provided, wherein the step (b) is processed by chromatographing, absorbing and separating jujuba cAMP of the first extract with the OU-2 macroporous resin bound with the aldehyde group.

### Method 14:

The above-mentioned preparation method is provided, wherein the step (b) further is processed by chromatographing, absorbing and separating jujuba cAMP of the first extract with the ME-2 macroporous resin bound with the aldehyde group.

### Method 15:

The pharmaceutical composition of the present invention can include the pharmacologically acceptable carriers, additives and the combination thereof.

### Method 16:

The pharmaceutical composition of the present invention can be manufactured as a dosage form, and the dosage forms is selected from any one of tablet, capsule, powder, pill, dust, solution, microcapsule, suspension, emulsion, particle, dropping pill, roll and the pharmacologically oral pharmaceutical dosage form.

### Method 17:

The pharmaceutical composition of the present invention can be manufactured as pharmaceuticals, health food and nutrient supplements for treating depression and anxiety disorder in accordance with the Good Manufacturing Practice (GMP) pharmaceutical standards and the method of health food producing/manufacturing standards.

### THE PREFERRED EMBODIMENT

The present invention is further illustrated as follows by combining the figures and the preferred embodiments.

### Embodiment 1:

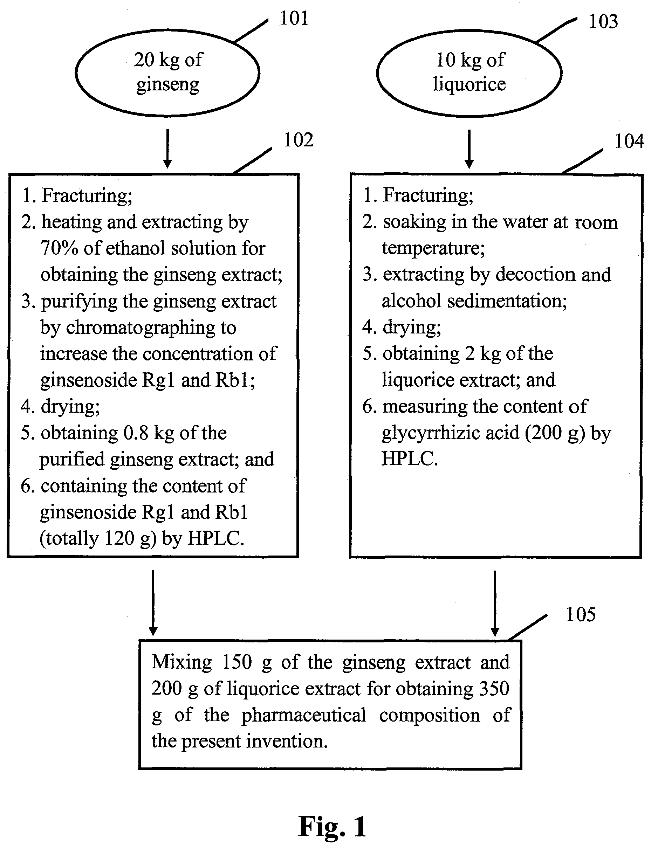

Please refer to Fig. 1, which is the flowchart showing a preparation method of a pharmaceutical composition in accordance with a first preferred embodiment of the present invention. In Fig. 1, after 20 kg of ginseng (101) is fractured, the fractured ginseng is heated to extract by 70% of the ethanol solution. The extracted ginseng is separated and purified by column chromatography, and dried, and 0.8 kg of the ginseng extract having 120 g of ginsenoside Rg1 and Rb1 is obtained (102). Further, after 10 kg of liquorice (103) is fractured, the fractured liquorice is soaked at room temperature for 12 hours. The soaked liquorice is extracted by decoction and alcohol sedimentation, concentrated and dried, and 2 kg of the liquorice extract having 200 g of glycyrrhizic acid is obtained (104). Afterwards, 150 g of the obtained ginseng extract and 200 g of the obtained liquorice extract are pulverized and mixed, and 350 g of the pharmaceutical composition (containing 22.5 g of ginsenoside Rg1 and Rb1, and 20 g of glycyrrhizic acid) of the present invention is obtained (105).

### Embodiment 2:

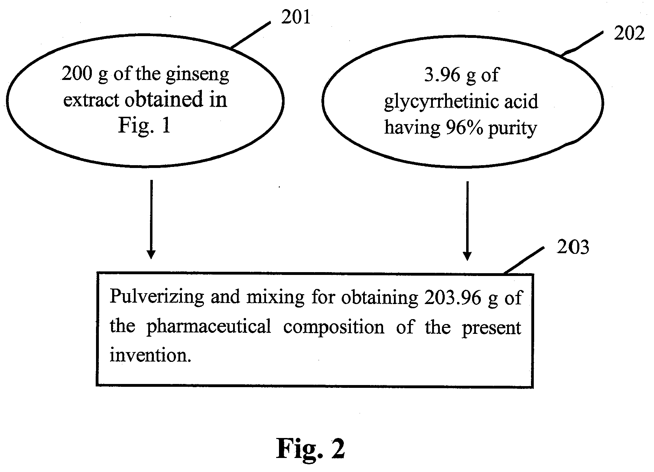

Please refer to Fig. 2, which is the flowchart showing a preparation method of a pharmaceutical composition in accordance with a second preferred embodiment of the present invention. In Fig. 2, after the prepared 3.96 g of glycyrrhetinic acid having 96% purity (202) and 200 g of the ginseng extract obtained in Embodiment 1 (201) are pulverized and mixed, 203.96 g of the pharmaceutical composition (containing 30 g of ginsenoside Rg1 and Rb1, and 3.8 g of glycyrrhetinic acid) of the present invention is obtained (203).

### Embodiment 3:

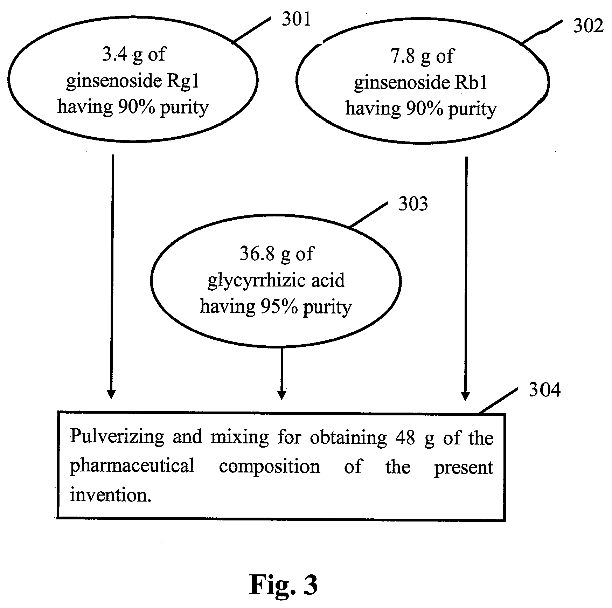

Please refer to Fig. 3, which is the flowchart showing a preparation method of a pharmaceutical composition in accordance with a third preferred embodiment of the present invention. In Fig. 3, after 3.4 g of the prepared ginsenoside Rg1 having 90% purity (301), 7.8 g of the prepared gensenoside Rb1 having 90% purity (302) and 36.8 g of glycyrrhizic acid having 90% purity (303) are pulverized and mixed, 48 g of the pharmaceutical composition (containing 10 g of ginsenoside Rg1 and Rb1, and 35 g of glycyrrhizic acid) of the present invention is obtained (304).

### Embodiment 4:

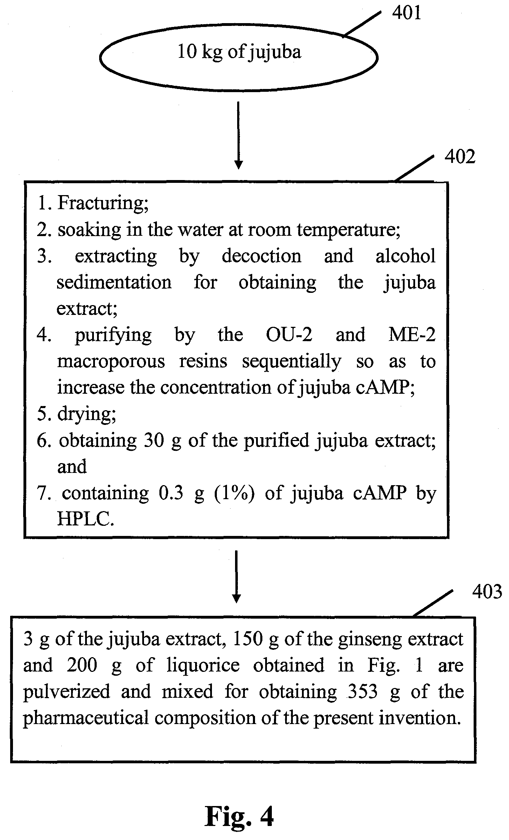

Please refer to Fig. 4, which is the flowchart showing a preparation method of a pharmaceutical composition in accordance with a fourth preferred embodiment of the present invention. In Fig. 4, 10 kg of jujuba (401) is fractured and soaked in the water at room temperature, then the soaked jujuba is extracted by decoction and alcohol sedimentation for obtaining the jujuba extract, which is further absorbed and separated by the OU-2 and ME-2 macroporous resins sequentially, and dried. Thirty (30) g of the jujuba extract containing 0.3 g of jujuba cAMP is obtained to be the raw material for preparing the pharmaceutical of the present invention (402).

Afterwards, after 150 g of the ginseng extract and 200 g of the liquorice extract obtained in Embodiment 1 are pulverized and mixed with 3 g of the above-mentioned jujuba extract, and 353 g of the pharmaceutical composition (containing 22.5 g of ginsenoside Rg1 and Rb1, 20 g of glycyrrhizic acid and 0.03 g of jujuba cAMP) of the present invention is obtained (403).

### Embodiment 5:

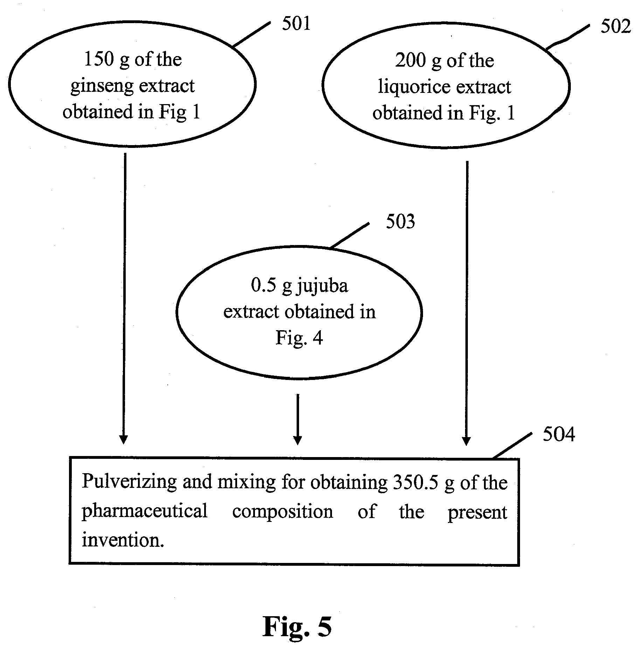

Please refer to Fig. 5, which is the flowchart showing a preparation method of a pharmaceutical composition in accordance with a fifth preferred embodiment of the present invention. In Fig. 5, after 150 g of the ginseng extract (501) and 200 g of the liquorice extract (502) obtained in Embodiment 1 respectively are pulverized and mixed with 0.5 g of the jujuba extract obtained in Embodiment 4 (503), 350.5 g of the pharmaceutical composition (containing 22.5 g of ginsenoside Rg1 and Rb1, 20 g of glycyrrhizic acid and 0.005 g of jujuba cAMP) of the present invention is obtained (504).

### Embodiment 6:

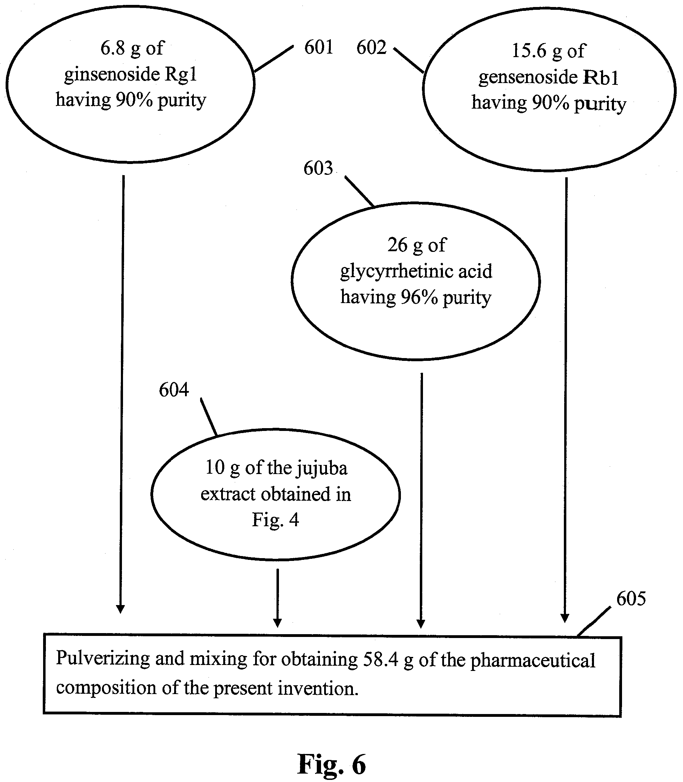

Please refer to Fig. 6, which is the flowchart showing a preparation method of a pharmaceutical composition in accordance with a sixth preferred embodiment of the present invention. In Fig. 6, after 6.8 g of the prepared ginsenoside Rg1 having 90% purity (601), 15.6 g of the prepared ginsenoside Rb1 having 90% purity (602), 26 g of glycyrrhetinic acid having 96% purity (603) and 10 g of the jujuba extract obtained in Embodiment 4 (604) are pulverized and mixed, 58.4 g of the pharmaceutical composition (containing 20 g of ginsenoside Rg1 and Rb1, 25 g of glycyrrhetinic acid and 0.1 g of jujuba cAMP) of the present invention is obtained (605).

### Experiment 1: The influence of Embodiment 1 in the mouse tail-hanging experiment

1.1 Experimental animals: ICR mice, male, 22.0±2 g of body weight, secondary, are provided by the Experimental Animal Science Department of Capital Medical University, Beijing.

1.2 Experimental pharmaceuticals: The pharmaceutical of Embodiment 1 is provided by Beijing Wonner Biotech. Ltd. Co., and Paroxetine (Paxil) is the product of Zhong Mei Tianjin Smith Kline pharmaceuticals Co. Ltd.

### 1.3 Experimental equipment: Stop watch.

1.4 Dose designs: 1. High dose of Embodiment 1 (80 mg/kg/d); 2. middle dose of Embodiment 1 (40 mg/kg/d); and 3. low dose of Embodiment 1 (20 mg/kg/d).

### 1.5 Experimental method and result:

1.5.1 Group division and administration of drug: The mice are grouped randomly, and 10 mice are in each group. 1. High dose of Embodiment 1 (80 mg/kg, per oral (P.O.), administered for 7 days); 2. middle dose of Embodiment 1 (40 mg/kg, P.O., administered for 7 days); 3. low dose of Embodiment 1 (20 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

1.5.2 Experimental method: The mouse's tail (1 cm to the tail end) is taped on the wood strip higher than the platform for 5 cm and hung up for 6 minutes. The time of non-movement of the mouse for the last 5 minutes is recorded.

1.5.3 Statistic calculation: The experimental data are represented as *X̅* ± *SD,* and the experimental result is calculated as analysis of variance (ANOVA) by SPSS 11.5 statistic software.

1.5.4 Experimental result: Please refer to Table 1.

**Table 1. The influence of Embodiment 1 on the time of non-movement of the mouse**

| Group | Animal number | Time of non-movement (s) |
|---|---|---|
| Physiological saline (control) | 10 | 113.22±21.18 |
| Paroxetine | 10 | 75.33±22.91* |
| High dose of Embodiment 1 | 10 | 54.67±26.38** |
| Middle dose of Embodiment 1 | 10 | 72.68±27.06* |
| Low dose of Embodiment 1 | 10 | 95.26±49.91 |

| | | |
|---|---|---|
| In comparison with the control group: *P < 0.05, and **P < 0.01. | | |

Conclusion: According to the above experiment, it can be found that the high and middle doses of Embodiment 1 of the present invention and Paroxetine all decreased the time of non-movement after the mouse's tail is hung, significantly different from the physiological group (control). Therefore, the Embodiment 1 of the present invention having anti-experimental depression function can be extrapolated.

### Experiment 2: The influence of Embodiment 1 in the Resetpine-induced mouse body temperature decrease experiment

2.1 Experimental animals: ICR mice, male, 22.0±2 g of body weight, secondary, are provided by the Experimental Animal Science Department of Capital Medical University, Beijing.

2.2 Experimental pharmaceuticals: The pharmaceutical of Embodiment 1 is provided by Beijing Wonner Biotech. Ltd. Co., Paroxetine (Paxil) is the product of Zhong Mei Tianjin Smith Kline pharmaceuticals Co. Ltd., and Resetpine is the product of Guangdong BangMin Pharmaceutical Co., Ltd.

2.3 Experimental equipments: Electronic thermometer (Model: GM222) and stop watch.

2.4 Dose designs: 1. High dose of Embodiment 1 (80 mg/kg/d); 2. middle dose of Embodiment 1 (40 mg/kg/d); and 3. low dose of Embodiment 1 (20 mg/kg/d).

### 2.5 Experimental method and result:

2.5.1 Group division and administration of drug: The mice are grouped randomly, and 10 mice are in each group. 1. High dose of Embodiment 1 (80 mg/kg, P.O., administered for 7 days); 2. middle dose of Embodiment 1 (40 mg/kg, P.O., administered for 7 days); 3. low dose of Embodiment 1 (20 mg/kg, P.O., administered for 7 days); 4. Paroxetine (3 mg/kg, P.O., administered for 7 days); and 5. physiological saline (P.O.).

2.5.2 Experimental method: After 1 hour of the last administration of drug on the eighth day, the mouse's anal temperature is determined. Then 2 mg of Resetpine per kilogram of the body weight is given by intraperitoneal injection. After 4 hours of injecting Resetpine, mouse's anal temperature is determined once again. The depth and time of injecting the thermometer into mouse's anus are identical in each temperature measurement.

2.5.3 Statistic calculation: The experimental data are represented as *X̅* ± *SD,* and the experimental result is calculated as ANOVA by SPSS 11.5 statistic software.

2.5.4 Experimental result: Please refer to Table 2.

**Table 2. The influence of Embodiment 1 on the decreased mouse body temperature induced by Resetpine**

| Group | Animal number | Decreased temperature (°C) |
|---|---|---|
| Physiological saline (control) | 10 | 3.65±0.77 |
| Paroxetine | 10 | 2.38±0.69** |
| High dose of Embodiment 1 | 10 | 1.85±1.01** |
| Middle dose of Embodiment 1 | 10 | 2.05±1.03** |
| Low dose of Embodiment 1 | 10 | 2.35±0.69** |

| | | |
|---|---|---|
| In comparison with the control group: *P < 0.05, and **P < 0.01. | | |

Conclusion: According to the above experiment, it can be found that the high, middle and low doses of Embodiment 1 of the present invention and Paroxetine all decreased the reduced body temperature induced by Resetpine, and it means that those anti-experimental depression functions might be related to and affected the amount of monoamine neurotransmitter. Therefore, the Embodiment 1 of the present invention having anti-experimental depression function can be extrapolated.

### Experiment 3: The influence of Embodiment 1 in the mouse light-dark transition experiment

3.1 Experimental animals: Kunming (KM) mice, male, 24 ~ 26 g of body weight, secondary, are provided by the Experimental Animal Science Department of Capital Medical University, Beijing.

3.2 Experimental pharmaceuticals: The pharmaceutical of Embodiment 1 is provided by Beijing Wonner Biotech. Ltd. Co., and Diazepam is the product of Tianjin Jinhuei Amino Acid Co. Ltd.

3.3 Experimental equipment: Self-made light-dark transition chamber.

3.4 Dose designs: 1. High dose of Embodiment 1 (80 mg/kg/d); 2. middle dose of Embodiment 1 (40 mg/kg/d); and 3. low dose of Embodiment 1 (20 mg/kg/d).

### 3.5 Experimental method and result:

3.5.1 Group division and administration of drug: The mice are grouped randomly as 5 groups, and 10 mice are in each group. 1. High dose of Embodiment 1 (80 mg/kg); 2. middle dose of Embodiment 1 (40 mg/kg); 3. low dose of Embodiment 1 (20 mg/kg); 4. Diazepam (2.5 mg/kg); and 5. physiological saline (normal saline, NS). The drugs are fed into the mouse stomach once every day, and the mouse is administered for continuous 7 days. In the period of administration, the mouse eats and drinks freely, and the experiment is proceeded after 1 hour of the last administration of drug on the eighth day.

### 3.5.2 Experimental method:

Mouse light-dark transition test: The dark chamber occupies one third of the light-dark transition chamber (44 cm × 21 cm × 21 cm), and the top is capped. The light chamber occupies two third thereof and is illuminated brightly. A door between two chambers is disposed for the passage of the mouse. The mouse is placed in the center of the light chamber when the test begins, and the mouse's back faces the dark chamber. The times that the mouse enters into the dark chamber and returns to the light chamber within 10 minutes are determined, and the times thereof are being the index for evaluating the anti-anxiety function of the drugs.

3.5.3 Statistic calculation: The experimental data are represented as *X̅* ± *SD,* and the experimental result is calculated as one-way ANOVA by SPSS 11.5 statistic software.

3.5.4 Experimental result: Please refer to Table 3.

**Table 3. The influence of Embodiment 1 on the times of the mouse light-dark transition test**

| Group | Animal number | Times of passing from dark chamber to light chamber |
|---|---|---|
| High dose of Embodiment 1 | 10 | 11.2±3.84* |
| Middle dose of Embodiment 1 | 10 | 13.1±5.38** |
| Low dose of Embodiment 1 | 10 | 13.5±4.65** |
| Diazepam | 10 | 11.3±4.54* |
| Physiological saline (control) | 10 | 6.2±4.32 |

| | | |
|---|---|---|
| In comparison with the control group: *P < 0.05, and **P < 0.01. | | |

3.6 Description: The light-dark transition test adopted in the present experiment is built up on the basis that the mouse congenitally hates the bright light and the voluntary exploring behavior to the new environment. The clinical pharmaceutical (Diazepam) for treating anxiety in human beings and the Embodiment 1 have excellent correlation on improving the function of the increasing voluntary exploring behavior of the mouse on this model. According to the above experiment, it can be found that the high, middle and low doses of Embodiment 1 of the present invention and Diazepam all significantly increase the times that the mouse passes from the dark chamber to the light chamber, and have statistically meanings while comparing with the physiological saline. The experimental result has proven that the Embodiment 1 has anti-anxiety function.

3.7 Conclusion: According to the above experimental result, the high, middle and low doses of Embodiment 1 of the present invention and Diazepam all significantly increase the times that the mouse passes from the dark chamber to the light chamber. The result represents the Embodiment 1 has anti-anxiety effect.

### Experiment 4: The influence of Embodiment 2 in the mouse tail-hanging experiment

4.1 Experimental animals: ICR mouse, male, 22.0±2 g of body weigh, secondary, are provided by the Experimental Animal Science Department of Capital Medical University, Beijing.

4.2 Experimental pharmaceuticals: The pharmaceutical of Embodiment 2 is provided by Beijing Wonner Biotech. Ltd. Co., and Paroxetine (Paxil) is the product of Zhong Mei Tianjin Smith Kline pharmaceuticals Co. Ltd.

### 4.3 Experimental equipment: Stop watch.

4.4 Dose designs: 1. High dose of Embodiment 2 (80 mg/kg/d); 2. middle dose of Embodiment 2 (40 mg/kg/d); and 3. low dose of Embodiment 2 (20 mg/kg/d).

### 4.5 Experimental method and result:

4.5.1 Group division and administration of drug: The mice are grouped randomly, and 10 mice are in each group. 1. High dose of Embodiment 2 (80 mg/kg, P.O., administered for 7 days); 2. middle dose of Embodiment 2 (40 mg/kg, P.O., administered for 7 days); 3. low dose of Embodiment 2 (20 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

4.5.2 Experimental method: The mouse's tail (1 cm to the tail end) is taped on the wood strip higher than the platform for 5 cm and hung up for 6 minutes. The time of non-movement of the mouse for the last 5 minutes is recorded.

4.5.3 Statistic calculation: The experimental data are represented as *X̅* ± *SD,* and the experimental result is calculated as ANOVA by SPSS 11.5 statistic software.

4.5.4 Experimental result: Please refer to Table 4.

**Table 4. The influence of Embodiment 2 on the decreased mouse body temperature induced by Resetpine**

| Group | Animal number | Time of non-movement (s) |
|---|---|---|
| Physiological saline (control) | 10 | 113.22±21.18 |
| Paroxetine | 10 | 75.33±22.91* |
| High dose of Embodiment 1 | 10 | 93.27±36.42 |
| Middle dose of Embodiment 1 | 10 | 76.21±28.36* |
| Low dose of Embodiment 1 | 10 | 107.79±32.56 |

| | | |
|---|---|---|
| In comparison with the control group: *P < 0.05, and **P < 0.01. | | |

Conclusion: According to the above experiment, it can be found that the middle dose of Embodiment 2 of the present invention and Paroxetine all decreased the time of non-movement after the mouse's tail is hung up, significantly different from the physiological group (control). Therefore, the Embodiment 2 of the present invention having anti-experimental depression function can be extrapolated.

### Experiment 5: The influence of Embodiment 2 in the Resetpine-induced decrease of mouse body temperature experiment

5.1 Experimental animals: ICR mice, male, 22.0±2 g of body weight, secondary, are provided by the Experimental Animal Science Department of Capital Medical University, Beijing.

5.2 Experimental pharmaceuticals: The pharmaceutical of Embodiment 2 is provided by Beijing Wonner Biotech. Ltd. Co., Paroxetine (Paxil) is the product of Zhong Mei Tianjin Smith Kline pharmaceuticals Co. Ltd., and Resetpine is the product of Guangdong BangMin Pharmaceutical Co., Ltd.

5.3 Experimental equipments: Electronic thermometer (Model: GM222) and stop watch.

5.4 Dose designs: 1. High dose of Embodiment 2 (80 mg/kg/d); 2. middle dose of Embodiment 2 (40 mg/kg/d); and 3. low dose of Embodiment 2 (20 mg/kg/d).

### 5.5 Experimental method and result:

5.5.1 Group division and administration of drug: The mice are grouped randomly, and 10 mice are in each group. 1. High dose of Embodiment 2 (80 mg/kg, P.O., administered for 7 days); 2. middle dose of Embodiment 2 (40 mg/kg, P.O., administered for 7 days); 3. low dose of Embodiment 2 (20 mg/kg, P.O., administered for 7 days); 4. Paroxetine (3 mg/kg, P.O., administered for 7 days); and 5. physiological saline (P.O.).

5.5.2 Experimental method: After 1 hour of the last administration of drug on the eighth day, the mouse's anal temperature is determined. Then 2 mg of Resetpine per kilogram of body weight is given by intraperitoneal injection. After 4 hours of injecting Resetpine, mouse's anal temperature is determined once again. The depth and time of injecting the thermometer into mouse's anus are identical in each temperature measurement.

5.5.3 Statistic calculation: The experimental data are represented as *X̅* ± *SD,* and the experimental result is calculated as ANOVA by SPSS 11.5 statistic software.

5.5.4 Experimental result: Please refer to Table 5.

**Table 5. The influence of Embodiment 2 on the decreased mouse body temperature induced by Resetpine**

| Group | Animal number | Time of non-movement (s) |
|---|---|---|
| Physiological saline (control) | 10 | 3.65±0.77 |
| Paroxetine | 10 | 2.38±0.69** |
| High dose of Embodiment 2 | 10 | 2.93±0.74* |
| Middle dose of Embodiment 2 | 10 | 2.31±0.82** |
| Low dose of Embodiment 2 | 10 | 3.21±0.71 |

| | | |
|---|---|---|
| In comparison with the control group: *P < 0.05, and **P < 0.01. | | |

Conclusion: According to the above experiment, it can be found that the middle dose of Embodiment 2 of the present invention and Paroxetine all decreased the reduced body temperature induced by Resetpine, and it means that those anti-experimental depression functions might be related to and affected the amount of monoamine neurotransmitter. Therefore, the Embodiment 2 of the present invention having anti-experimental depression function can be extrapolated.

### Experiment 6: The influence of Embodiment 3 in the mouse tail-hanging experiment

6.1 Experimental animals: ICR mice, male, 22.0±2 g of body weight, secondary, are provided by the Experimental Animal Science Department of Capital Medical University, Beijing.

6.2 Experimental pharmaceuticals: The pharmaceutical of Embodiment 3 is provided by Beijing Wonner Biotech. Ltd. Co., and Paroxetine (Paxil) is the product of Zhong Mei Tianjin Smith Kline pharmaceuticals Co. Ltd.

### 6.3 Experimental equipment: Stop watch.

6.4 Dose designs: 1. High dose of Embodiment 3 (80 mg/kg/d); 2. middle dose of Embodiment 3 (40 mg/kg/d); and 3. low dose of Embodiment 3 (20 mg/kg/d).

### 6.5 Experimental method and result:

6.5.1 Group division and administration of drug: The mice are grouped randomly, and 10 mice are in each group. 1. High dose of Embodiment 3 (80 mg/kg, P.O., administered for 7 days); 2. middle dose of Embodiment 3 (40 mg/kg, P.O., administered for 7 days); 3. low dose of Embodiment 3 (20 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

6.5.2 Experimental method: The mouse's tail (1 cm to the tail end) is taped on the wood strip higher than the platform for 5 cm and hung up for 6 minutes. The time of non-movement of the mouse for the last 5 minutes is recorded.

6.5.3 Statistic calculation: The experimental data are represented as *X̅* ± *SD,* and the experimental result is calculated as ANOVA by SPSS 11.5 statistic software.

6.5.4 Experimental result: Please refer to Table 6.

**Table 6. The influence of Embodiment 3 on the time of non-movement of the mouse**

| Group | Animal number | Time of non-movement (s) |
|---|---|---|
| Physiological saline (control) | 10 | 113.22±21.18 |
| Paroxetine | 10 | 75.33±22.91* |
| High dose of Embodiment 3 | 10 | 70.37±28.14* |
| Middle dose of Embodiment 3 | 10 | 76.26±23.81* |
| Low dose of Embodiment 3 | 10 | 90.40±31.32 |

| | | |
|---|---|---|
| In comparison with the control group: *P < 0.05, and **P < 0.01. | | |

Conclusion: According to the above experiment, it can be found that the high and middle doses of Embodiment 3 of the present invention and Paroxetine all decreased the time of non-movement after the mouse's tail is hung up, significantly different from the physiological group (control). Therefore, the Embodiment 3 of the present invention having anti-experimental depression function can be extrapolated.

### Experiment 7: The influence of Embodiment 3 in the Resetpine-induced mouse body temperature decrease experiment

7.1 Experimental animals: ICR mice, male, 22.0±2 g of body weight, secondary, are provided by the Experimental Animal Science Department of Capital Medical University, Beijing.

7.2 Experiment pharmaceuticals: The pharmaceutical of Embodiment 3 is provided by Beijing Wonner Biotech. Ltd. Co., Paroxetine (Paxil) is the product of Zhong Mei Tianjin Smith Kline pharmaceuticals Co. Ltd., and Resetpine is the product of Guangdong BangMin Pharmaceutical Co., Ltd.

7.3 Experimental Equipments: Electronic thermometer (Model: GM222) and stop watch.

7.4 Dose designs: 1. High dose of Embodiment 3 (80 mg/kg/d); 2. middle dose of Embodiment 3 (40 mg/kg/d); and 3. low dose of Embodiment 3 (20 mg/kg/d).

### 7.5 Experimental method and result:

7.5.1 Group division and administration of drug: The mice are grouped randomly, and 10 mice are in each group. 1. High dose of Embodiment 3 (80 mg/kg, P.O., administered for 7 days); 2. middle dose of Embodiment 3 (40 mg/kg, P.O., administered for 7 days); 3. low dose of Embodiment 3 (20 mg/kg, P.O., administered for 7 days); 4. Paroxetine (3 mg/kg, P.O., administered for 7 days); and 5. physiological saline (P.O.).

2.5.2 Experimental method: After 1 hour of the last administration of drug on the eighth day, the mouse's anal temperature is determined. Then 2 mg of Resetpine per kilogram of body weight is given by intraperitoneal injection. After 4 hours of injecting Resetpine, mouse's anal temperature is determined once again. The depth and time of injecting the thermometer into mouse's anus are identical in each temperature measurement.

7.5.3 Statistic calculation: The experimental data are represented as *X̅* ± *SD,* and the experimental result is calculated as ANOVA by SPSS 11.5 statistic software.

7.5.4 Experimental result: Please refer to Table 7.

**Table 7. The influence of Embodiment 3 on the decreased mouse body temperature induced by Resetpine**

| Group | Animal number | Decreased temperature (°C) |
|---|---|---|
| Physiological saline (control) | 10 | 3.65±0.77 |
| Paroxetine | 10 | 2.38±0.69** |
| High dose of Embodiment 3 | 10 | 2.18±0.92** |
| Middle dose of Embodiment 3 | 10 | 2.36±0.83** |
| Low dose of Embodiment 3 | 10 | 2.97±0.67* |

| | | |
|---|---|---|
| In comparison with the control group: *P < 0.05, and **P < 0.01. | | |

Conclusion: According to the above experiment, it can be found that the high, middle and low doses of Embodiment 3 of the present invention and Paroxetine all decreased the reduced body temperature induced by Resetpine, and it means that those anti-experimental depression functions might be related to and affected the amount of monoamine neurotransmitter. Therefore, the Embodiment 3 of the present invention having anti-experimental depression function can be extrapolated.

### Experiment 8: The influence of Embodiment 4 in the mouse olfactory bulb lesion experiment

### 8.1 Experimental animals:

Olfactory bulb lesion model: Health Wistar male rats, secondary, 330±20 g of body weight, are purchased from Beijing Vital River Experimental Animal Technology Ltd. Co. (The quality certificate number: SCXK (JING) 2002-2003).

7.2 Reagents and pharmaceuticals: The pharmaceutical of Embodiment 4 is provided by Beijing Wonner Biotech Ltd. Co. (Lot: 060313), and Paroxetine is the product of Zhong Mei Tianjin Smith Kline pharmaceuticals Co. Ltd. (Lot: 04050011). The above pharmaceuticals are prepared with 0.5% of sodium carboxymethylcellulose (CMC-Na) for feeding into the stomach. Benzylpenicillin sodium for injection is the product of North China Pharmaceutical Huasheng Co. Ltd. (Lot: S0511204), and norepinephrine (NE) and 5-hydrotryptamine (5-HT) standards are the products of Sigma Co.. Other reagents are all marketed.

8.3 Equipments: Self-made open field activity box, step through box, rat stereotaxic instrument, high performance liquid chromatography (HPLC), and 10-tube γ radiation immunity arithmometer (Mode: DFM-96).

### 8.4 Experimental methods:

8.4.1 Group division and administration of drug: The rats are grouped randomly into 6 groups. 1. False therapy group; 2. model group (control); 3. high dose of Embodiment 4 (60 mg/kg/d); 4. middle dose of Embodiment 4 (30 mg/kg/d); 5. low dose of Embodiment 4 (15 mg/kg/d); and 6. Paroxetine (2 mg/kg/d). The test drugs and the positive drug are prepared with 0.5% of CMC-Na for feeding into the stomach once every day.

8.4.2 Preparation method of Model: The rat is anesthetized by chloral hydrate. After anesthesia, the linea median of the rat's fontanel is carved from 1 cm prior to the anterior fontanel to 1 cm behind the anterior fontanel, and the ossa cranii is exposed. The skull windows having 2 mm of diameter are opened from 8 mm prior to the anterior fontanel and in 2 mm of two sides of the linea median. The specially made electric soldering iron is inserted perpendicularly into the skull for 2 seconds, and the olfactory bulb is destroyed. The hemostatic sponge is filled into the skull windows and the skin is sewn. After the therapy, 40,000 unit of benzylpenicillin sodium per kilogram of body weight is given by intraperitoneal injection for every four days, and the tested pharmaceutical is given continuously for 24 days.

### 8.5 Observation index:

8.5.1 Open field activity experiment: The open field activity box (1 m × 1 m × 0.4 m) is constructed by the light blue plywood and the aluminum alloy frame. The box bottom is separated into 25 grids (20 cm × 20 cm for each grid), the circumference is the peripheral grids (16 grids), and others are the central girds (9 grids). The rat is placed in the center of the central grids, and the rat's cross-grid number (the number of crossing into the neighboring gird more than 3 claws) and rat's standing number (two forelimbs leaving the ground for more than 1 cm) are calculated/observed within 3 minutes.

8.5.2 Passive avoidance experiment (Step-through test): The step through box is configured by the light chamber and the dark chamber, and a channel is linked between the light chamber and the dark chamber for mouse entrance and exit. The grille of the dark chamber is connected to the electric shock equipment, and a mobile plate is set there between. If the rat enters into the dark chamber, the rat will be electrically shocked. During training, the rat is placed in the light chamber and then back in the hole for adaptation for 5 minutes. Then the plate is removed and the rat is observed for another 5 minutes. The time that the rat enters for the first time is recorded (electric-shocking latent period), and the time thereof is the learning record. After 24 hours, the test is repeated. The plate is removed and the dark chamber is electrified for 5 minutes so as to observe the time that the rat enters into the dark chamber for the first time. The time thereof is the memory record.

8.6 Statistic calculation: The experimental data are represented as *X̅* ± *SD,* and the experimental result is calculated as ANOVA by SPSS 11.5 statistic software.

### 8.7 Experimental results:

8.7.1 The result of the open field activity experiment: Please refer to Table 8.

**Table 8. The result of the rat olfactory bulb lesion model in the open field activity experiment**

| Group | Animal number | Horizontal movement (cross-grid number) | Vertical movement (standing number) |
|---|---|---|---|
| High dose of Embodiment 4 | 11 | 49.18±27.68** | 10.91±6.91** |
| Middle dose of Embodiment 4 | 11 | 54.55±23.01 | 13.45±5.72* |
| Low dose of Embodiment 4 | 11 | 61.82±21.43 | 15.18±4.47 |
| Paroxetine | 11 | 55.36±25.96* | 14.36±5.55 |
| Model group (control) | 11 | 79.55±24.33 | 19.09±8.53 |
| False therapy group | 11 | 45.36±26.84** | 10.45±6.19** |

| | | | |
|---|---|---|---|
| In comparison with the control group: *P < 0.05, and **P < 0.01. | | | |

8.7.2 The result of the step through test: Please refer to Table 9.

**Table 9. The result of the rat olfactory bulb lesion model in the step through test**

| Group | Animal number | Latent period of the 1st day (s) | Latent period of the 2nd day (s) |
|---|---|---|---|
| High dose of Embodiment 4 | 11 | 187.00±87.59* | 289.82±28.59* |
| Middle dose of Embodiment 4 | 11 | 191.71±72.95* | 288.82±37.09* |
| Low dose of Embodiment 4 | 11 | 152.44±76.81 | 271.18±38.61 |
| Paroxetine | 11 | 181.87±90.54* | 265.00±65.39 |
| Model group (control) | 11 | 111.21±82.93 | 236.88±74.17 |
| False therapy group | 11 | 211.46±82.10** | 292.82±14.37* |

| | | | |
|---|---|---|---|
| In comparison with the control group: *P < 0.05, and **P < 0.01. | | | |

Conclusion: The result of Experiment 8 shows that the high dose of Embodiment 4 can obviously improve the increase of the rat's horizontal and vertical movements caused by the olfactory bulb lesion, and the middle dose of Embodiment 4 also has obvious improved function on the increase of the vertical movement caused by the rat olfactory bulb lesion model. In addition, the high and middle doses of Embodiment 4 have obvious improved effects on the decreases of the rat study and memory function caused by the olfactory bulb lesion.

### Experiment 9: The influence of Embodiment 4 in the rat unpredictable long-term stimulus experiment

### 9.1 Experimental animals:

Unpredictable long-term stimulus model: Health Wistar male rats, secondary, 240 ~ 270 g of body weight, are purchased from Beijing Vital River Experimental Animal Technology Ltd. Co. (The quality certification number: SCXK (JING) 2002-2003).

9.2 Reagents and pharmaceuticals: The pharmaceutical of Embodiment 4 is provided by Beijing Wonner Biotech Ltd. Co. (Lot: 060313), and Paroxetine is the product of Zhong Mei Tianjin Smith Kline pharmaceuticals Co. Ltd. (Lot: 04050011). The above pharmaceuticals are prepared with 0.5% of sodium carboxymethylcellulose (CMC-Na) for feeding into the stomach. Benzylpenicillin sodium for injection is the product of North China Pharmaceutical Huasheng Co. Ltd. (Lot: S0511204), and norepinephrine (NE) and 5-hydrotryptamine (5-HT) standards are the products of Sigma Co. Other reagents are all marketed.

9.3 Equipments: self-made open field activity box, step through box, rat stereotaxic instrument, high performance liquid chromatography (HPLC), and 10-tribe γ radiation immunity arithmometer (Mode: DFM-96).

### 9.4 Experimental method:

9.4.1 Group division and administration of drug: The rats are grouped randomly into 6 groups. 1. False therapy group; 2. model group (control); 3. high dose of Embodiment 4 (60 mg/kg/d); 4. middle dose of Embodiment 4 (30 mg/kg/d); 5. low dose of Embodiment 4 (15 mg/kg/d); and 6. Paroxetine (2 mg/kg/d). The test drugs and the positive drug are prepared with 0.5% of CMC-Na for feeding into the stomach once every day.

### 9.4.2 Preparation method of Model:

Unpredictable long-term stimulus model: The rats in the control group eat and drink normally, and there is no stimulus performed. In other 5 groups, one rat is fed in each cage, and the rat is subject to the 24-day unpredictable stress/stimulus, including 24-hour abstinence for 3 times, 24-hour without drinking for 3 times, 24-hour wet bedding for 3 times (200 ml of water is added in the rat cage), overnight illumination for 3 times, swimming at 4°C for 5 minutes for 3 times, heating at 45°C in the oven for 5 minutes for 3 times, clipping the rat tail for 1 minute for 3 times, and 30-minute highspeed horizontal shake for 3 times. One stimulus is performed randomly every day and a total of 24 days. Each stimulus cannot be performed continuously. The pharmaceutical is fed into the rat's stomach once every day and a total of 24 days.

### 9.5 Observation index:

9.5.1 Open field activity experiment: Same as above.

9.5.2 Passive avoidance experiment: Same as above.

9.5.3 Rat swimming by compulsion test: This experiment is proceeded for two days after the last administration of drug. On the first day, the experiment is pre-performed for 15 minutes. The water temperature in the glass tank is 25°C, and the depth of water is 25 cm. After 24 hours, the formal experiment is proceeded. After 1 hour of administration of drug, the rat is placed in the tank, and the time of non-movement of the rat for the last 5 minutes is recorded.

9.5.4 Body weight measurement: The increasing values before and after each animal experiment are compared.

9.5.5 Volume test of drinking sucrose: The sucrose-intake volume of the rats are compared. The rats in each group drink 1% of the sucrose for 1 hour. The drinking volumes are determined before the stimulus and after 3 weeks of the stimulus. After the rat is in abstinence and water for 14 hours, 1% of the sucrose is placed in the cage and substituted for the original drinking water. The differences of the bottle weight before and after the rat drinking sucrose for 1 hour are measured and recorded, and the sucrose-drinking volume for each time is calculated. The difference of the sucrose-intake volume for each time is compared.

9.5.6 HPLC-electrochemical test: The amounts of norepinephrine and 5-hydrotryptamine in the rat cerebral cortex are measured.

9.6 Statistic calculation: The experimental data are represented as *X̅* ± *SD,* and the experimental result is calculated as ANOVA by SPSS 11.5 statistic software.

### 9.7 Experimental result:

9.7.1 Volume test of drinking sucrose: Please refer to Table 10.

**Table 10. The sucrose-intake volume of the rat in the unpredictable long-term stimulus model**

| Group | Animal number | Sucrose-intake volume (g) |
|---|---|---|
| High dose of Embodiment 4 | 13 | 22.55±5.03 |
| Middle dose of Embodiment 4 | 13 | 26.53±4.99** |
| Low dose of Embodiment 4 | 13 | 26.97±6.93** |
| Paroxetine | 13 | 26.29±4.87** |
| Model group (control) | 13 | 19.42±4.25 |
| False therapy group | 13 | 29.41±3.83** |

| | | |
|---|---|---|
| In comparison with the control group: *P < 0.05, and **P < 0.01. | | |

9.7.2 The result of the increasing rat body weight: Please refer to Table 11.

**Table 11. The increasing rat body weight in the unpredictable long-term stimulus model**

| Group | Animal number | Time of non-movement (s) |
|---|---|---|
| High dose of Embodiment 4 | 13 | 86.08±10.84 |
| Middle dose of Embodiment 4 | 13 | 96.00±11.05** |
| Low dose of Embodiment 4 | 13 | 95.15±15.46** |
| Paroxetine | 13 | 96.85±9.30** |
| Model group (control) | 13 | 84.92±12.61 |
| False therapy group | 13 | 120.54±10.60** |

| | | |
|---|---|---|
| In comparison with the control group: *P < 0.05, and **P < 0.01. | | |

9.7.3 The time of non-movement of the rat swimming by compulsion: Please refer to Table 12.

**Table 12. The time of non-movement in the unpredictable long-term stimulus model of the rat swimming by compulsion test**

| Group | Animal number | Time of non-movement (s) |
|---|---|---|
| High dose of Embodiment 4 | 13 | 23.28±18.05** |
| Middle dose of Embodiment 4 | 13 | 18.95±12.55** |
| Low dose of Embodiment 4 | 13 | 25.20±13.60* |
| Paroxetine | 13 | 31.38±19.59 |
| Model group (control) | 13 | 39.95±17.46 |
| False therapy group | 13 | 26.96±12.76* |

| | | |
|---|---|---|
| In comparison with the control group: *P < 0.05, and **P < 0.01. | | |

9.7.4 The result of the open field activity experiment: Please refer to Table 13.

**Table 13. The result of the unpredictable long-term stimulus model in the open field activity experiment**

| | Animal number | Horizontal movement (cross-grid number) | Vertical movement (standing number) |
|---|---|---|---|
| High dose of Embodiment 4 | 14 | 58.31±15.35* | 16.54±4.24** |
| Middle dose of Embodiment 4 | 14 | 49.15±14.26 | 13.54±4.48 |
| Low dose of Embodiment 4 | 14 | 52.62±21.83 | 16.15±7.32* |
| Paroxetine | 14 | 61.85±21.68** | 14.69±4.2 |
| Model group (control) | 14 | 39.54±16.31 | 11.46±3.26 |
| False therapy group | 14 | 64.00±11.97** | 16.85±3.18** |

| | | | |
|---|---|---|---|
| In comparison with the control group: *P < 0.05, and **P < 0.01. | | | |

9.7.5 The result of the rat passive avoidance experiment: Please refer to Table 14.

**Table 14. The result of the unpredictable long-term stimulus model in the rat passive avoidance experiment**

| | Animal number | Latent period of first day (s) | Latent period of second day (s) |
|---|---|---|---|
| High dose of Embodiment 4 | 13 | 65.43±31.44 | 259.22±56.51 |
| Middle dose of Embodiment 4 | 13 | 58.18±18.0 | 212.76+77.27 |
| Low dose of Embodiment 4 | 13 | 75.98±32.22* | 204.85±94.99 |
| Paroxetine | 13 | 75.75±46.52* | 257.46±66.92 |
| Model group (control) | 13 | 50.01±15.7 | 189.25±111.99 |
| False therapy group | 13 | 80.00±39.17* | 263.38±59.68 |

| | | | |
|---|---|---|---|
| In comparison with the control group: *P < 0.05, and **P < 0.01. | | | |

9.7.6 The result of NE and 5-HT contents in the rat cerebral cortex: Please refer to Table 14.

**Table 14. The result of NE and 5-HT contents in the rat cerebral cortex in the unpredictable long-term stimulus model**

| | Animal number | 5-HT (nmol/L brain tissue extracts) | NE (nmol/L brain tissue extracts) |
|---|---|---|---|
| High dose of Embodiment 4 | 12 | 230.4157±47.78554* | 269.5409±58.86389** |
| Middle dose of Embodiment 4 | 12 | 303.4418±70.31711** | 227.2976±28.95101** |
| Low dose of Embodiment 4 | 12 | 332.7343±76.25168** | 201.8688±29.80775** |
| Paroxetine | 12 | 227.0637±46.53838* | 220.5419±38.31681** |
| Model group (control) | 12 | 179.3866±20.49374 | 57.6671±77.66958 |
| False therapy group | 12 | 228.1478±28.40397* | 132.8598±20.84756** |

| | | | |
|---|---|---|---|
| In comparison with the control group: *P < 0.05, and **P < 0.01. | | | |

Conclusion: The results of Experiment 9 are shown as follows. The middle and low doses of Embodiment 4 can obviously improve the decreased sucrose-drinking volume and the decreased body weight in the unpredictable long-term stress/stimulus. The high, middle and low doses of Embodiment 4 all obviously increase the time of non-movement in the rat swimming by compulsion test. The high dose of Embodiment 4 can obviously improve the decreased rat horizontal and vertical movements caused by the unpredictable long-term stress/stimulus. The low dose of Embodiment 4 also has obvious improved function on the decreased rat vertical movement caused by the unpredictable long-term stress/stimulus. The low dose of Embodiment 4 has the improved function on the decreased rat learning ability caused by the unpredictable long-term stress/stimulus. The high, middle and low doses of Embodiment 4 all obviously increase NE and 5-HT contents in the rat cerebral cortex.

### Experiment 10: The influence of Embodiment 4 in the mouse light-dark transition experiment

10.1 Experimental animals: Kunming (KM) mice, male, 24 ~ 26 g of body weight, secondary, are provided by the Experimental Animal Science Department of Capital Medical University, Beijing.

10.2 Experimental pharmaceuticals: The pharmaceutical of Embodiment 4 is provided by Beijing Wonner Biotech. Ltd. Co., and Diazepam is the product of Tianjin Jinhuei Amino Acid Co. Ltd.

10.3 Experimental equipment: Self-made light-dark transition box.

10.4 Dose designs: 1. High dose of Embodiment 4 (80 mg/kg/d); 2. middle dose of Embodiment 4 (40 mg/kg/d); and 3. low dose of Embodiment 4 (20 mg/kg/d).

### 10.5 Experimental method and result:

10.5.1 Group division and administration of drug: The mice are grouped randomly as 5 groups, and 10 mice are in each group. 1. High dose of Embodiment 4 (80 mg/kg); 2. middle dose of Embodiment 4 (40 mg/kg); 3. low dose of Embodiment 4 (20 mg/kg); 4. Diazepam (2.5 mg/kg); and 5. physiological saline (normal saline, NS). The drugs are fed into the mouse stomach once every day, and the mouse is administered for continuous 7 days. In the period of administration, the mouse eats and drinks freely, and the experiment is proceeded after 1 hour of the last administration of drug on the eighth day.

### 10.5.2 Experimental method:

Mouse light-dark transition test: The dark chamber occupies one third of the light-dark transition chamber (44 cm × 21 cm × 21 cm), and the top is capped. The light chamber occupies two third thereof and is illuminated brightly. A door between two chambers is disposed for the passage of the mouse. The mouse is placed in the center of the light chamber when the experiment begins, and the mouse's back faces the dark chamber. The times that the mouse enters into the dark chamber and returns to the light chamber within 10 minutes are determined, and the times thereof are being the index for evaluating the anti-anxiety function of the drugs.

10.5.3 Statistic calculation: The experimental data are represented as *X̅* ± *SD*, and the experimental result is calculated as one-way ANOVA by SPSS 11.5 statistic software.

10.5.4 Experimental result: Please refer to Table 16.

**Table 16. The influence of Embodiment 4 in the times of the mouse light-dark transition experiment**

| Group | Animal number | Times of passing from dark chamber to light chamber |
|---|---|---|
| High dose of Embodiment 4 | 10 | 11.6±4.53* |
| Middle dose of Embodiment 4 | 10 | 13.9±3.76** |
| Low dose of Embodiment 4 | 10 | 13.4±4.12** |
| Diazepam | 10 | 11.7±4.47* |
| Physiological saline (control) | 10 | 6.8±3.85 |

| | | |
|---|---|---|
| In comparison with the control group: *P < 0.05, and **P < 0.01. | | |

10.6 Description: The light-dark transition experiment adopted in the present experiment is built up on the basis that the mouse congenitally hates the bright light and the voluntary exploring behavior to the new environment. The clinical pharmaceutical (Diazepam) for treating anxiety in human beings and the Embodiment 4 have excellent correlation on improving the function of the increasing voluntary exploring behavior in the mouse model. According to the above experiment, it can be found that the high, middle and low doses of Embodiment 4 of the present invention and Diazepam all significantly increase the times that the mouse passes from the dark chamber to the light chamber, and have statistically meanings while comparing with the normal saline. The experimental result has proven that the Embodiment 4 has anti-anxiety effect.

10.7 Conclusion: According to the above experimental result, the high, middle and low doses of Embodiment 4 of the present invention and Diazepam all significantly increase the times that the mouse passes from the dark chamber to the light chamber. The result represents the Embodiment 4 has anti-anxiety ability.

### Experiment 11: The influence of Embodiment 5 in the mouse tail-hanging experiment

11.1 Experiment pharmaceuticals: The pharmaceutical of Embodiment 5 is provided by Beijing Wonner Biotech. Ltd. Co. (pilot magnification product), and Paroxetine is the product of Zhong Mei Tianjin Smith Kline pharmaceuticals Co. Ltd. (Lot: 05070384). Above-mentioned pharmaceuticals are prepared with physiological saline for feeding into the stomach.

11.2 Experimental animals: ICR mice, male, 20.0±1 g of body weight, secondary, are provided by the Experimental Animal Science Department of Capital Medical University, Beijing. The quality certification number of mice is SCXK (JING) 2006-2008.

### 11.3 Experimental equipment: Stop watch.

11.4 Method: Seventy (70) mice are randomly grouped for 5 groups: normal saline (NS) group, Paroxetine (3 mg/kg/d), high dose of Embodiment 5 (80 mg/kg/d), middle dose of Embodiment 5 (40 mg/kg/d), and low dose of Embodiment 5 (20 mg/kg/d). The pharmaceuticals are fed into mouse's stomach once every day. After 1 hour of the last administration of drug on the eighth day, the mouse's tail (1 cm to the tail end) is taped on the horizontal support in an opened box, and the mouse represents the reversed hung status. The mouse head is away from the bottom of the opened box for about 10 cm, and the mouse is hung up for 6 minutes. The time of non-movement of the mouse for the last 5 minutes is recorded.

11.5 Statistic calculation: The experimental data are represented as *X̅* ± SD, and the experimental result is calculated as one-way ANOVA by SPSS 11.5 statistic software.

11.6 Experimental result: The result of the time of non-movement of the mouse tail-hanging experiment: Please refer to Table 17.

**Table 17. The influence of Embodiment 5 on the accumulative time of non-movement of the mouse**

| Group | Animal number | Dose (mg/kg/d) | Time of non-movement(s) |
|---|---|---|---|
| High dose of Embodiment 5 | 14 | 80 | 64.75±42.22** |
| Middle dose of Embodiment 5 | 14 | 40 | 55.41±33.83** |
| Low dose of Embodiment 5 | 14 | 20 | 62.75±26.61** |
| Paroxetine | 14 | 3 | 53.27±20.02** |
| Normal saline (NS) group | 14 | | 113.59±36.11 |

| | | | |
|---|---|---|---|
| In comparison with the NS group: *P < 0.05, and **P < 0.01. | | | |

Conclusion: The research result shows that the high, middle and low doses of Embodiment 5 and the clinical effective anti-depression pharmaceutical, Paroxetine, all obviously decrease the accumulative time of non-movement of hanging mouse's tail. It represents that Embodiment 5 of the present invention has anti-experimental depression ability.

### Experiment 12: The influence of Embodiment 5 in the mouse swimming by compulsion experiment

12.1 Experiment pharmaceuticals: The pharmaceutical of Embodiment 5 is provided by Beijing Wonner Biotech. Ltd. Co. (pilot magnification product), and Paroxetine is the product of Zhong Mei Tianjin Smith Kline pharmaceuticals Co. Ltd. (Lot: 05070384). The above-mentioned pharmaceuticals are prepared with physiological saline for feeding into the stomach.

12.2 Experimental animals: ICR mice, male, 20.0±1 g of body weight, secondary, are provided by the Experimental Animal Science Department of Capital Medical University, Beijing. The quality certification number of mice is SCXK (JING) 2006-2008.

### 12.3 Experimental equipment: Stop watch.

12.4 Experimental method: The mouse group and the administration of drugs are identical with the mouse tail-hanging experiment. The tested mouse in each group is proceeded the experiment after 1 hour of the administration of drug. The mouse is trained to swim for 15 minutes before the experiment and on the eighth day. After 24 hours, the experiment is performed. The mouse is placed in 25°C water in the glass tank having 10 cm of water depth, 14 cm of diameter. The accumulative time of non-movement of the mouse for the last 5 minutes is recorded.

12.5 Statistic calculation: The experimental data are represented as *X̅* ± *SD,* and the experimental result is calculated as one-way ANOVA by SPSS 11.5 statistic software.

12.6 Experiment result: The result of the mouse swimming by compulsion: Please refer to Table 18.

**Table 18. The influence of Embodiment 5 on the time of the mouse swimming by compulsion test**

| Group | Animal number | Dose (mg/kg/d) | Time of non-movement (s) |
|---|---|---|---|
| High dose of Embodiment 5 | 14 | 80 | 88.35±51.64* |
| Middle dose of Embodiment 5 | 14 | 40 | 65.87±38.96** |
| Low dose of Embodiment 5 | 14 | 20 | 88.12±38.57* |
| Paroxetine | 14 | 3 | 57.80±38.07** |
| Normal saline (NS) group | 14 | | 132.47±40.64 |

| | | | |
|---|---|---|---|
| **I**n comparison with the NS group: *P < 0.05, and **P < 0.01. | | | |

Conclusion: The research result shows that the high, middle and low doses of Embodiment 5 and the clinical effective anti-depression pharmaceutical, Paroxetine, all obviously decrease the accumulative time of non-movement of the mouse swimming by compulsion test. It represents that Embodiment 5 of the present invention has anti-experimental depression ability.

### Experiment 13:

Nine (9) kg of the remained ginseng debris and 7 kg of the remained liquorice debris extracted from Embodiment 1 and Embodiment 4 are collected, dried, pulverized, and mixed well for obtaining the debris mixture containing the trace amounts of ginsenoside Rg1 and Rb1, glycyrrhizic acid and jujuba cAMP. The control experiment of influence in the mouse tail-hanging experiment is proceeded.

13.1 Experimental animals: ICR mice, male, 22.0±2 g of body weight, secondary, are provided by the Experimental Animal Science Department of Capital Medical University, Beijing.

13.2 Experiment pharmaceuticals: The debris mixture is provided by Beijing Wonner Biotech. Ltd. Co., and Paroxetine (Paxil) is the product of Zhong Mei Tianjin Smith Kline pharmaceuticals Co. Ltd.

### 13.3 Experimental equipment: Stop watch.

13.4 Dose designs: 1. High dose of debris mixture (160 mg/kg/d); 2. middle dose of debris mixture (80 mg/kg/d); and 3. low dose of debris mixture (40 mg/kg/d).

### 13.5 Experimental method and result:

13.5.1 Group division and administration of drug: The mice are grouped randomly, and 10 mice are in each group. 1. High dose of debris mixture (160 mg/kg, P.O., administered for 7 days); 2. middle dose of debris mixture (80 mg/kg, P.O., administered for 7 days); 3. low dose of debris mixture (40 mg/kg, P.O., administered for 7 days); 4. Paroxetine (3 mg/kg, P.O., administered for 7 days); and 5. physiological saline (P.O.). After 1 hour of the last administration of drug, the mouse tail-hanging experiment is proceeded.

13.5.2 Experimental method: The mouse's tail (1 cm to the tail end) is taped on the wood strip higher than the platform for 5 cm and hung up for 6 minutes. The time of non-movement of the mouse for the last 5 minutes is recorded.

13.5.3 Statistic calculation: The experimental data are represented as *X̅* ± *SD*, and the experimental result is calculated as ANOVA by SPSS 11.5 statistic software.

13.5.4 Experimental result: Please refer to Table 19.

**Table 19. The influence of debris mixture on the time of non-movement of the mouse**

| Group | Animal number | Time of non-movement (s) |
|---|---|---|
| Physiological saline (control) | 10 | 82.03±43.01 |
| Paroxetine | 10 | 38.37±20.76* |
| High dose of debris mixture | 10 | 76.91±31.09 |
| Middle dose of debris mixture | 10 | 78.89±48.18 |
| Low dose of debris mixture | 10 | 81.31±58.68 |

| | | |
|---|---|---|
| In comparison with the control group: *P < 0.05, and **P < 0.01. | | |

Conclusion: According to the above experiment, it can be found that although the high, middle and low doses of the debris mixture can shorten the time of non-movement of the mouse tail-hanging, these three groups have no significance comparing with the physiological saline (control). Therefore, the debris mixture without anti-experimental depression function can be extrapolated.

### Industrial usefulness:

The application scopes of the pharmaceutical composition of the present invention for treating depression and anxiety disorder lie in that:
1. the described pharmaceutical composition of the present invention for treating depression and anxiety disorder can include the pharmacologically acceptable addictives;
2. the described pharmaceutical composition of the present invention for treating depression and anxiety disorder can be manufactured as the known dosage forms, such as powder, capsule and tablet, etc.; and
3. the described pharmaceutical composition of the present invention for treating depression and anxiety disorder can be manufactured as the health food for treating depression and anxiety disorder.

## Claims

1. A pharmaceutical composition for use in the treatment of a depression and an anxiety disorder, **characterized by** comprising:
2 ~ 25 parts by weight of a ginsenoside having an Rg1 and an Rb1; and
3 ~ 46 parts by weight of a glycyrrhizically related acid selected from the group consisting of glycyrrhizic acid, glycyrrhetinic acid and a combination thereof.

2. The pharmaceutical composition for use according to claim 1 **characterized by** comprising 4 ~ 12 parts by weight of the ginsenoside and 5 ~ 15 parts by weight of the glycyrrhizically related acid.

3. The pharmaceutical composition for use according to claim 1 further **characterized by** comprising jujuba cyclic adenosine monophosphate (jujuba cAMP).

4. The pharmaceutical composition for use according to claim 3 **characterized by** comprising 0.002 ~ 0.4 parts by weight of the jujuba cAMP, and preferably **characterized by** comprising 0.01 ~ 0.08 parts by weight of the jujuba cAMP.

5. The pharmaceutical composition for use according to claim 3, **characterized in that** the ginsenoside is extracted from a ginseng, the glycyrrhizically related acid is extracted from a liquorice, and the jujuba cAMP is extracted from a jujuba.

6. The pharmaceutical composition for use according to claim 3, **characterized in that** the jujuba is extracted for obtaining a first extract having a first jujuba cAMP concentration, the first extract is further extracted for obtaining a second extract having a second jujuba cAMP concentration, the second jujuba cAMP concentration is higher than the first jujuba cAMP concentration, and the second extract is a raw material in the pharmaceutical composition.

7. A pharmaceutical composition for use in the treatment of a depression and an anxiety disorder, **characterized by** comprising 4 ~ 60 parts by weight of a ginseng and 2 ~ 30 parts by weight of a liquorice.

8. The pharmaceutical composition for use according to claim 7 **characterized by** comprising 10 ~ 28 parts by weight of the ginseng and 5 ~ 14 parts by weight of the liquorice.

9. The pharmaceutical composition for use according to claim 7 further **characterized by** comprising a jujuba.

10. The pharmaceutical composition for use according to claim 9 **characterized by** comprising 2 ~ 40 parts by weight of the jujuba, and preferably **characterized by** comprising 4 ~ 18 parts by weight of the jujuba.

11. The pharmaceutical composition for use according to claim 7 further **characterized by** comprising at least one of a pharmacologically acceptable carrier and an additive, having a dosage form selected from a group consisting of a tablet, a capsule, a powder, a pill, a dust, a solution, a microcapsule, a suspension, an emulsion, a particle, a dropping pill and a roll, and being manufactured as one of a health food and a nutrient supplement.

12. A pharmaceutical composition, comprising 2 ~ 25 parts by weight of a ginsenoside having an Rg1 and an Rb1, and 3 ~ 46 parts by weight of a glycyrrhizically related acid selected from the group consisting of glycyrrhizic acid, glycyrrhetinic acid and a combination thereof.

13. The pharmaceutical composition according to Claim 12, wherein the pharmaceutical composition is further **characterized by** comprising jujuba cyclic adenosine monophosphate (jujuba cAMP).

14. The pharmaceutical composition according to Claim 12, wherein the pharmaceutical composition is manufactured as one selected from the group consisting of a pharmaceutical, a health food and a nutrient.

## Patentansprüche

1. Pharmazeutische Zusammensetzung zur Verwendung in der Behandlung einer Depression und einer Angststörung, **dadurch gekennzeichnet, dass** sie Folgendes enthält: 2-25 Gewichtsteile eines Ginsenosids Rg1 und Rb1; und 3-46 Gewichtsteile einer glycyrrhizinartigen Säure, die aus der Gruppe ausgewählt ist, die aus Glycyrrhizinsäure, Glycyrrhetinsäure und einer Kombination davon besteht.

2. Pharmazeutische Zusammensetzung zur Verwendung nach Anspruch 1, **dadurch gekennzeichnet, dass** sie 4-12 Gewichtsteile des Ginsenosids und 5-15 Gewichtsteile der glycyrrhizinartigen Säure enthält.

3. Pharmazeutische Zusammensetzung zur Verwendung nach Anspruch 1, die weiterhin **dadurch gekennzeichnet ist, dass** sie zyklisches Adenosin-Monophosphat der Jujube (Jujuben-cAMP) enthält.

4. Pharmazeutische Zusammensetzung zur Verwendung nach Anspruch 3, **dadurch gekennzeichnet, dass** sie 0,002-0,4 Gewichtsteile der Jujuben-cAMP enthält, und vorzugsweise **dadurch gekennzeichnet, dass** sie 0,01 ~ 0,08 Gewichtsteile der Jujuben-cAMP enthält.

5. Pharmazeutische Zusammensetzung zur Verwendung nach Anspruch 3, **dadurch gekennzeichnet, dass** das Ginsenosid aus einem Ginsengprodukt extrahiert wird, die glycyrrhizinartige Säure aus einem Lakritzprodukt extrahiert wird und die Jujuben-cAMP aus einem Jujubenprodukt extrahiert wird.

6. Pharmazeutische Zusammensetzung zur Verwendung nach Anspruch 3, **dadurch gekennzeichnet, dass** das Jujubenprodukt eine Extraktion erfährt, um einen ersten Extrakt mit einer ersten Konzentration an Jujuben-cAMP zu erhalten, der erste Extrakt weiter extrahiert wird, um einen zweiten Extrakt mit einer zweiten Konzentration an Jujuben-cAMP zu erhalten, die zweite Konzentration an Jujuben-cAMP höher als die erste Konzentration an Jujuben-cAMP ist, und es sich bei dem zweiten Extrakt um einen Rohstoff in der pharmazeutischen Zusammensetzung handelt.

7. Pharmazeutische Zusammensetzung zur Verwendung in der Behandlung einer Depression und einer Angststörung, **dadurch gekennzeichnet, dass** sie 4-60 Gewichtsteile eines Ginsengprodukts und 2-30 Gewichtsteile eines Lakritzprodukts enthält.

8. Pharmazeutische Zusammensetzung zur Verwendung nach Anspruch 7, **dadurch gekennzeichnet, dass** sie 10-28 Gewichtsteile des Ginsengprodukts und 5-14 Gewichtsteile des Lakritzprodukts enthält.

9. Pharmazeutische Zusammensetzung zur Verwendung nach Anspruch 7, weiterhin **dadurch gekennzeichnet, dass** sie ein Jujubenprodukt enthält.

10. Pharmazeutische Zusammensetzung zur Verwendung nach Anspruch 9, **dadurch gekennzeichnet, dass** sie 2-40 Gewichtsteile des Jujubenprodukts enthält, und vorzugsweise **dadurch gekennzeichnet, dass** sie 4-18 Gewichtsteile des Jujubenprodukts enthält.

11. Pharmazeutische Zusammensetzung zur Verwendung nach Anspruch 7, weiterhin **dadurch gekennzeichnet, dass** sie mindestens einen pharmakologisch unbedenklichen Trägerstoff und einen Zusatzstoff enthält, wobei sie eine Darreichungsform aufweist, die aus einer Gruppe ausgewählt ist, die aus einer Tablette, einer Kapsel, einem Pulver, einer Pille, einem Feinpulver, einer Lösung, einer Mikrokapsel, einer Suspension, einer Emulsion, einem Partikel, einer Schmelzsuspensionspille und einer Rolle besteht, und wobei sie derart hergestellt wird, dass sie entweder ein gesundheitsförderndes Lebensmittel oder ein nährstoffliefernden Zusatz ist.

12. Pharmazeutische Zusammensetzung, die 2-25 Gewichtsteile eines Ginsenosids Rg1 und Rb1 enthält sowie 3 ~ 46 Gewichtsteile einer glycyrrhizinartigen Säure, die aus der Gruppe ausgewählt ist, die aus Glycyrrhizinsäure, Glycyrrhetinsäure und einer Kombination davon besteht.

13. Pharmazeutische Zusammensetzung nach Anspruch 12, wobei die pharmazeutische Zusammensetzung weiterhin **dadurch gekennzeichnet ist, dass** sie zyklisches Adenosin-Monophosphat der Jujube (Jujuben-cAMP) enthält.

14. Pharmazeutische Zusammensetzung nach Anspruch 12, wobei die pharmazeutische Zusammensetzung derart hergestellt wird, dass sie ein Element aus der Gruppe bildet, die aus einem pharmazeutischen Mittel, einem gesundheitsfördernden Lebensmittel und einem Nährstoff besteht.

## Revendications

1. Composition pharmaceutique destinée à être utilisée dans le traitement d'un trouble lié à une dépression et à une anxiété, **caractérisée en ce qu'**elle comprend :
de 2 à environ 25 parties en poids d'un ginsénoside Rg1 et Rb1 ; et
de 3 à environ 46 parties en poids d'un acide de la famille de l'acide glycyrrhizique choisi dans le groupe formé par l'acide glycyrrhizique, l'acide glycyrrhétinique et une combinaison de ceux-ci.

2. Composition pharmaceutique destinée à être utilisée selon la revendication 1, **caractérisée en ce qu'**elle comprend de 4 à environ 12 parties en poids de ginsénoside et de 5 à environ 15 parties en poids de l'acide de la famille de l'acide glycyrrhizique.

3. Composition pharmaceutique destinée à être utilisée selon la revendication 1, **caractérisée en outre en ce qu'**elle comprend de l'adénosine monophosphate cyclique de jujube (AMPc de jujube).

4. Composition pharmaceutique destinée à être utilisée selon la revendication 3, **caractérisée en ce qu'**elle comprend de 0,002 à environ 0,4 parties en poids d'AMPc de jujube, et de préférence **caractérisée en ce qu'**elle comprend de 0,01 à environ 0,08 parties en poids d'AMPc de jujube.

5. Composition pharmaceutique destinée à être utilisée selon la revendication 3, **caractérisée en ce que** le ginsénoside est extrait d'un ginseng, l'acide de la famille de l'acide glycyrrhizique est extrait de la réglisse, et l'AMPc du jujube est extrait du jujube.

6. Composition pharmaceutique destinée à être utilisée selon la revendication 3, **caractérisée en ce que** le jujube est extrait pour obtenir un premier extrait avec une première concentration en AMPc de jujube, le premier extrait est extrait une nouvelle fois pour obtenir un second extrait ayant une seconde concentration en AMPc de jujube, la seconde concentration en AMPc de jujube est plus élevée que la première concentration en AMPc de jujube, et le second extrait est une matière première de la composition pharmaceutique.

7. Composition pharmaceutique destinée à être utilisée dans le traitement d'un trouble lié à une dépression et à une anxiété, **caractérisée en ce qu'**elle comprend de 4 à environ 60 parties en poids de ginseng et de 2 à environ 30 parties en poids de réglisse.

8. Composition pharmaceutique destinée à être utilisée selon la revendication 7, **caractérisée en ce qu'**elle comprend de 10 à environ 28 parties en poids de ginseng et de 5 à environ 14 parties en poids de réglisse.

9. Composition pharmaceutique destinée à être utilisée selon la revendication 7, **caractérisée en ce qu'**elle comprend en outre du jujube.

10. Composition pharmaceutique destinée à être utilisée selon la revendication 9, **caractérisée en ce qu'**elle comprend de 2 à environ 40 parties en poids de jujube, et de préférence **caractérisée en ce qu'**elle comprend de 4 à environ 18 parties en poids de jujube.

11. Composition pharmaceutique destinée à être utilisée selon la revendication 7, **caractérisée en outre en ce qu'**elle comprend au moins un vecteur pharmacologiquement acceptable et un additif, ayant une forme galénique choisie dans le groupe constitué par un comprimé, une gélule, une poudre, une pilule, une poudre fine, une solution, une microgélule, une émulsion, une particule, un comprimé à avaler et un produit compacté avec un rouleau et qui est fabriquée sous une forme d'aliment diététique et de complément alimentaire.

12. Composition pharmaceutique, comprenant de 2 à environ 25 parties en poids d'un ginsénoside Rg1 et Rb1, et de 3 à environ 46 parties en poids d'un acide de la famille de l'acide glycyrrhizique choisi dans le groupe formé par l'acide glycyrrhizique, l'acide glycyrrhétinique et une combinaison de ceux-ci.

13. Composition pharmaceutique selon la revendication 12, où la composition pharmaceutique est en outre **caractérisée en ce qu'**elle comprend de l'adénosine monophosphate cyclique de jujube (AMPc de jujube).

14. Composition pharmaceutique selon la revendication 12, où la composition pharmaceutique est fabriquée en tant qu'une composition choisie dans le groupe constitué par un produit pharmaceutique, un aliment diététique et un complément alimentaire.
